# EUROPEAN PATENT APPLICATION

(11) **EP 4 538 707 A1**
(43) Date of publication of application: **16.04.2025**
(21) Application number: 23383037.1
(22) Date of filing: 09.10.2023
(51) Int. Cl.: G01N 33/68

(54) **SNFL AND SGFAP LEVELS AS PREDICTIVE BIOMARKERS FOR TREATMENT RESPONSE IN RELAPSING MS PATIENTS TREATED WITH OCRELIZUMAB**

(71) Applicant: Fundación para la Investigación Biomédica del Hospital Universitario Ramón y Cajal, 28034 Madrid (ES)
(72) Inventor: VILLAR GUIMERANS, María Luisa, 28034 Madrid (ES); COSTA-FROSSARD FRANÇA, Lucienne, 28034 Madrid (ES); MONREAL LAGUILLO, Enric, 28034 Madrid (ES); SAINZ DE LA MAZA CANTERO, Susana, 28034 Madrid (ES); CHICO GARCÍA, Juan Luís, 28034 Madrid (ES); RODRÍGUEZ JORGE, Fernando, 28034 Madrid (ES); FERNÁNDEZ VELASCO, José Ignacio, 28034 Madrid (ES); VILLARRUBIA MIGALLÓN, Noelia, 28034 Madrid (ES); ESPIÑO MARTÍNEZ, María Mercedes, 28034 Madrid (ES)
(74) Representative: Hoffmann Eitle

(57) **Abstract**

The invention relates to a method of predicting a subject's response to anti-CD20 antibody, preferably ocrelizumab, therapy.

## Description

### TECHNICAL FIELD

The invention relates to a method of predicting a subject's response to anti-CD20 antibody, preferably ocrelizumab, therapy.

### BACKGROUND ART

Multiple sclerosis (MS) is a chronic autoimmune disease of the central nervous system (CNS), characterized by inflammation, demyelination, and axonal damage, which leads to the deterioration of neurological function. The accumulation of irreversible clinical disability can result from either relapse-associated worsening (RAW) or progression independent of relapse activity (PIRA). High-efficacy disease-modifying therapies (heDMTs), such as anti-CD20 monoclonal antibodies, have demonstrated nearly complete suppression of relapses and the formation of new or enlarged T2-weighted magnetic resonance imaging (MRI) lesions. However, a significant percentage of patients experience silent disability progression. Serum neurofilament light chain (sNfL) levels, obtained within the first year of the disease, have emerged as a useful prognostic biomarker capable of identifying MS patients at high risk of disability progression. Early administration of heDMTs in this patient group may prevent such outcomes. Conversely, elevated serum glial fibrillary acidic protein (sGFAP) levels appear to be associated with PIRA. The impact of heDMTs on patients with high sGFAP values is yet to be determined. Ocrelizumab (Ocrevus^{®}, Hoffmann-La Roche, Grenzach-Wyhlen, Germany) is a humanized monoclonal antibody that specifically targets CD20+ B cells. It has demonstrated a high level of efficacy in preventing new relapses and T2 MRI lesions, with an overall favourable safety profile in clinical trials. Furthermore, ocrelizumab has shown a lower progression rate than interferon beta-1a, suggesting its potential role in preventing PIRA, particularly in patients with higher exposure to the drug. It has been suggested that higher levels of ocrelizumab in the serum may have a greater impact on disability progression. However, there is a lack of predictive factors for clinical response to ocrelizumab in relapsing-remitting MS. The present invention provides sNfL and sGFAP levels as predictive biomarkers for treatment response in relapsing MS patients treated with ocrelizumab.

### BRIEF DESCRIPTION OF THE FIGURES

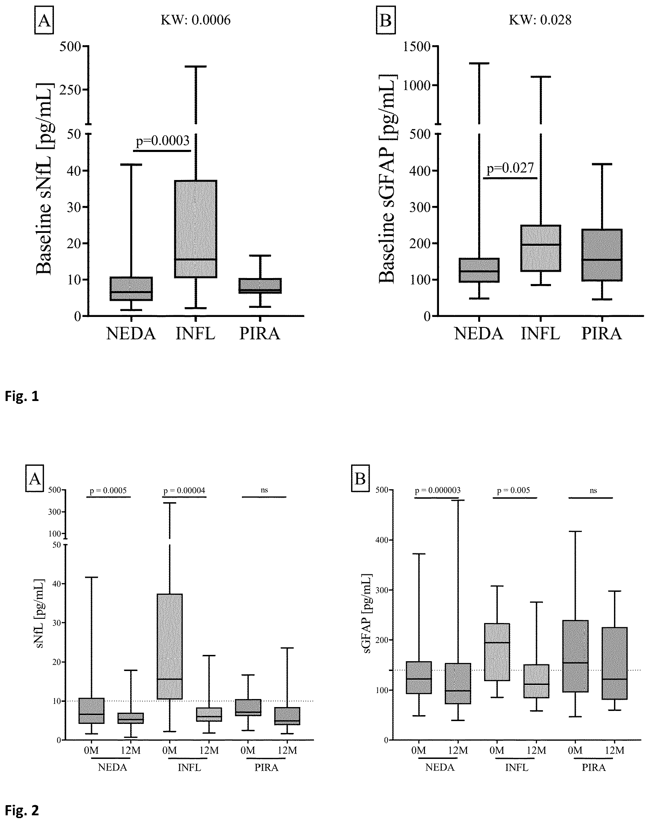

**Figure** 1. Differences between three groups of RRMS patients in baseline sNfL [A] and sGFAP [B] levels. Abbreviations: RRMS, relapsing-remitting multiple sclerosis; sNfL, serum neurofilament light chains; sGFAP, serum glial fibrillary acidic protein. KW: Kruskal Wallis test. NEDA: patients who reached no evidence of disease activity (NEDA)-3 status at 12 months of ocrelizumab initiation (n=71); INFL: patients who had relapses and/or radiological activity but no disability progression at 12 months of ocrelizumab initiation (n=19); PIRA: patients who had confirmed disability progression without inflammatory activity at 12 months of ocrelizumab initiation (n=10).
**Figure 2****.** Changes in sNfL [A] and sGFAP [B] levels induced by ocrelizumab. sNfL and sGFAP levels measured before (0M) and at 12 months (12M) of ocrelizumab initiation. Abbreviations: sNfL, serum neurofilament light chains; sGFAP, serum glial fibrillary acidic protein. NEDA: patients who reached no evidence of disease activity (NEDA)-3 status at 12 months of ocrelizumab initiation (n=71); INFL: patients who had relapses and/or radiological activity but no disability progression at 12 months of ocrelizumab initiation (n=19); PIRA: patients who had confirmed disability progression without inflammatory activity at 12 months of ocrelizumab initiation (n=10).

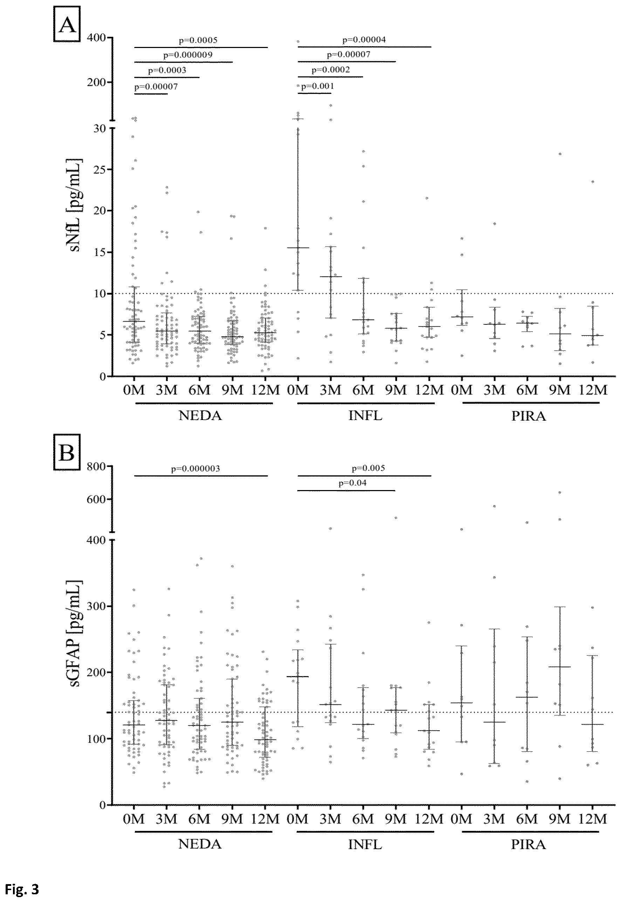

**Figure 3****.** Three month variations in sNfL [A] and sGFAP [B] levels induced by ocrelizumab treatment. sNfL and sGFAP levels measured before (0M) and at 3 (3M), 6 (6M), 9 (9M) and 12 (12M) months of ocrelizumab initiation. Abbreviations: sNfL, serum neurofilament light chains; sGFAP, serum glial fibrillary acidic protein. NEDA: patients who reached no evidence of disease activity (NEDA)-3 status at 12 months of ocrelizumab initiation (n=71); INFL: patients who had relapses and/or radiological activity but no disability progression at 12 months of ocrelizumab initiation (n=19); PIRA: patients who had confirmed disability progression without inflammatory activity at 12 months of ocrelizumab initiation (n=10).

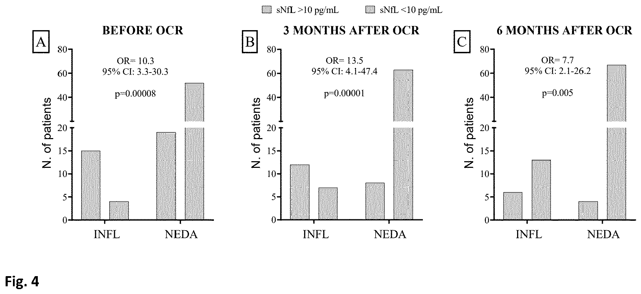

**Figure 4****.** Risk of achieving inflammatory activity at baseline [A], three [B] and six [C] months of ocrelizumab follow-up. Number (N) of patients with sNFL values above (orange) or below (green) 10 pg/mL Abbreviations: sNfL, serum neurofilament light chains; NEDA: patients who reached no evidence of disease activity (NEDA)-3 status at 12 months of ocrelizumab initiation (n=71); INFL: patients who had relapses and/or radiological activity but no disability progression at 12 months of ocrelizumab initiation (n=19). OR, odds ratio; CI: confidence interval.

### DEFINITIONS

The terms "treatment" and "therapy", as used in the present application, refer to a set of hygienic, pharmacological, surgical and/or physical means used with the intent to cure and/or alleviate a disease and/or symptoms with the goal of remediating the health problem. The terms "treatment" and therapy" include preventive and curative methods, since both are directed to the maintenance and/or reestablishment of the health of an individual or animal. Regardless of the origin of the symptoms, disease and disability, the administration of a suitable medicament to alleviate and/or cure a health problem should be interpreted as a form of treatment or therapy within the context of this application.

The terms "individual", "patient" or "subject" are used interchangeably in the present application and are not meant to be limiting in any way. The "individual", "patient" or "subject" can be of any age, sex and physical condition. Preferably, these terms refer to a human patient.

The terms "Multiple Sclerosis" or "MS" refer to a chronic inflammatory disease of the central nervous system (CNS) of unknown etiology, in which immune cells migrate to the central nervous system inducing demyelination and axonal damage.

The terms "relapse remitting Multiple Sclerosis" or "RRMS" refer to a type of MS characterized by flare-ups or relapses. The flare-ups or relapses may occur after a period of inactivity of the disease

The term "isolated blood sample" refers to any blood sample which has been previously obtained, i.e. isolated, from a patient. It is to be understood that the act of obtaining the blood samples from a patient is not an aspect of the present invention.

The term "ocrelizumab" is a humanised anti-CD20 monoclonal antibody developed for the treatment of multiple sclerosis (MS). It was approved by the Food and Drug Administration (FDA) in March 2017 for using in adults with relapsing-remitting multiple sclerosis (RRMS) and primary progressive multiple sclerosis (PPMS). Ocrelizumab is the only disease-modifying therapy (DMT) approved for PPMS. In November 2017, the European Medicines Agency (EMA) also approved ocrelizumab as the first drug for people with early PPMS. (Hauser SL, et al. Ocrelizumab versus interferon beta-1a in relapsing multiple sclerosis. N. Engl. J. Med. 2017;376:221-234. doi: 10.1056/NEJMoa1601277).

The term "sNfL" shall be understood as serum neurofilament light chains levels.

The term "sGFAP" shall be understood as serum glial fibrillary acidic protein levels.

### DESCRIPTION OF EMBODIMENTS

Ocrelizumab is a high-efficacy monoclonal antibody that selectively depletes CD20+ cells while maintaining B-cell reconstitution and pre-existing humoral immunity. We aimed to determine whether serum NfL or GFAP values could predict disease progression or inflammatory activity in a multicenter prospective cohort of 100 RRMS patients treated with this B-cell-depleting treatment. After 1 year of ocrelizumab treatment, the majority of patients achieved NEDA, while a low percentage of patients experienced inflammation, which was related to higher levels of sNFL. A low percentage of patients showed PIRA, associated with low levels of sNfL and high levels of sGFAP. NfL levels reflect acute axonal damage and are strongly associated with acute inflammatory activity, manifested as relapses or new MRI lesions. They can serve as a biomarker for monitoring inflammation and disease progression, particularly when combined with elevated levels of sGFAP, a marker of astroglial activation.

Our objective was to investigate the role of both biomarkers in determining the response to ocrelizumab in relapsing-remitting MS. We initially examined baseline sNfL values in our three patient groups. As higher levels appear to increased risk of inflammation and disease progression, we established a cut off value of 10 pg/mL. Elevated levels of sNfL indicated patients at risk of experiencing inflammatory disease activity. Furthermore, the rate at which sNfL values decreased proved to be an even better indicator of treatment response. Most NEDA patients exhibited normalized sNfL values after 3 months, whereas in patients with ongoing inflammation, levels remained above 10 pg/mL until around nine months into ocrelizumab treatment before normalizing. This decrease was associated with clinical outcomes, as relapses were predominantly observed within the first 6-month period.

Thus, high sNfL levels at the 3-month mark could serve as a reliable biomarker for identifying patients at a high risk of inflammation. This information could be valuable in determining which patients would benefit from a higher dose of ocrelizumab, as it has been suggested to be beneficial in a subgroup of MS patients that is not yet precisely defined. In contrast, PIRA patients exhibited low sNfL values at the start of treatment and did not experience any significant changes during the follow-up period. Inflammation was also associated with high sGFAP levels. Elevated levels of this variable, particularly when combined with high sNfL values, indicated patients at a high risk of disease progression. However, this association was not observed in patients treated with ocrelizumab. In fact, ocrelizumab could reduce sGFAP values in both NEDA and INFL patients. This reduction followed the pattern observed in sNfL but with a delayed timeframe. This suggests that control of the acute inflammatory response precedes the normalization of the innate immune response, which has been closely linked to neurodegeneration.

The elevated values of both sNfL and sGFAP could be associated with inflammatory PIRA, which occurs in the early stages of the disease and can contribute to the worsening of the EDSS score independently of relapses. This strongly indicates that the onset of relapses alone is insufficient to detect patient deterioration associated with inflammation. High sNfL and sGFAP levels can serve as accurate biomarkers to identify additional patients at risk of EDSS worsening due to acute inflammation. Therefore, the term RAW could potentially be replaced with "AIAW" (acute inflammation-associated worsening) to include patients with relapses or those with biomarkers indicating inflammation-associated progression. Similarly, the definition of PIRA could be changed to "PIAI" (progression independent of acute inflammation). These definitions are important as they more accurately describe two types of progression in MS, which may imply different responses to treatment. Thus, while ocrelizumab may counteract AIAW, it does not appear to be effective in PIAI patients. This distinction should aid in fine-tuning treatment selection for MS. Future research is needed to determine the effects of other disease-modifying drugs in preventing both types of disease progression. On the other hand, a good strategy to avoid PIAI could be to make early treatment decisions. In our study, patients with PIRA had longer disease duration and higher baseline EDSS scores. All of them were previously on oral drugs, and only two had sNfL values above 10 pg/mL, compared to 60% who had elevated sGFAP values unaffected by ocrelizumab treatment. A more inflammatory phase could precede PIRA in these patients and allowing suboptimal response to treatment for prolonged periods could result in a highly disabling disease with fewer therapeutic options. The biomarkers described here could help prevent therapeutic inertia, which affects up to 25% of daily treatment decisions in MS. There is currently no standardized criterion for establishing a cut-off value for sGFAP levels. While a zeta score has been defined to account for age and sex effects on sGFAP values, other studies have used raw data to determine normal values. In our study, we established a cut-off value of 140 pg/mL.

The present invention thus provides, in a first aspect, a method for monitoring or predicting the risk of disease activity derived from the treatment with an anti-CD20 antibody, preferably ocrelizumab, in a patient who suffers from Multiple Sclerosis, preferably from relapse remitting Multiple Sclerosis, comprising the steps of:
(a) quantifying NfL and GFAP levels from an isolated blood or serum sample taken from the patient,
(b) comparing said NfL and GFAP levels of step a) with reference values or reference scores, wherein said corresponding reference (value or score) is linked to disease activity in patients having been treated with the anti-CD20 antibody, preferably ocrelizumab, and
(c) identifying the patient as:
   (i) patients at risk of having relapses and/or radiological activity but no disability progression at 12 months of the anti-CD20 antibody, preferably ocrelizumab, treatment initiation if
      (i.1) the sample of step a) is obtained from the patient before treatment, and the NfL and GFAP levels are higher than the reference value/s or reference score/s; and/or if
      (i.2) the isolated blood or serum sample is obtained from the patient after the anti-CD20 antibody, preferably ocrelizumab, treatment, preferably after 1, 2, 3, 6, 7, 8, 9, 10, 11, or 12 months after the first anti-CD20 antibody, preferably ocrelizumab, cycle, preferably about 3 months after the first anti-CD20 antibody, preferably ocrelizumab, cycle, and the NfL levels of step a) when compared with a reference value or reference score, are lower, preferably statistically significantly lower, than the reference value or score. It is noted that in this case, said reference value or reference score is calculated from a sample obtained from the patient before treatment (i.e., also referred herein, as baseline). More preferably, in this case the levels of NfL in the isolated blood or serum sample obtained from the patient before treatment (i.e., also referred herein, as baseline) are greater than 10 pg/mL;
   (ii) patients who reached no evidence of disease activity (NEDA) status at 12 months of the anti-CD20 antibody, preferably ocrelizumab, treatment initiation, if criteria (i.2) is fulfilled and the levels of NfL in an isolated blood or serum sample obtained from the patient before treatment (i.e., also referred herein, as baseline) are below 10 pg/mL;
   (iii) patients at risk of disability progression without inflammatory activity at 12 months of the anti-CD20 antibody, preferably ocrelizumab, treatment initiation if the criteria indicated in (i) or (ii) is not fulfilled.

It is noted that disability progression is measured in accordance with the Expanded Disability Status Scale (EDSS) score. It is further noted that, as indicated in the examples of the present invention, Ocrelizumab treatment is deemed to be especially effective in those patients pertaining to groups (i) and (ii). That is, while ocrelizumab may aid patients with relapses or those with biomarkers indicating inflammation-associated progression, it does not appear to be effective in patients having a progression independent of acute inflammation. This distinction should aid in fine-tuning treatment selection for MS.

In a preferred embodiment the reference value/s or reference score/s of (i. 1) are 10 pg/mL for sNfL and 140 pg/mL for sGFAP. That is, if the sample of step a), obtained from the patient before treatment, comprises sNfL levels greater than 10 pg/mL and sGFAP levels greater than 140 pg/mL, then the patient is classified as a patient at risk of having relapses and/or radiological activity but no disability progression at 12 months of ocrelizumab initiation.

The present invention additionally provides, in a second aspect, a method for predicting a treatment outcome with an anti-CD20 antibody, preferably ocrelizumab, in a patient who suffers from Multiple Sclerosis, comprising the steps of:
(a) quantifying NfL and/or GFAP levels from an isolated blood or serum sample taken from the patient, and
(b) comparing said NfL and/or GFAP levels of step a) with a reference value or reference score,
   wherein said corresponding reference (value or score) is linked to a treatment outcome in patients having been treated with the anti-CD20 antibody, preferably ocrelizumab, and
   wherein the deviation between the NfL and/or GFAP levels of step a) with the score or value of reference derived from the comparison step b) is used to predict a treatment outcome for said patient with Multiple Sclerosis when treated with the anti-CD20 antibody, preferably ocrelizumab.

In a preferred embodiment, the method is for predicting a treatment outcome with the anti-CD20 antibody, preferably ocrelizumab, in a patient who suffers from Multiple Sclerosis, comprises the steps of:
(a) quantifying NfL and GFAP levels from an isolated blood or serum sample taken from the patient, and
(b) comparing said NfL and GFAP levels of step a) with reference values or reference scores,
   wherein said corresponding references (value or score) are linked to a treatment outcome in patients having been treated with the anti-CD20 antibody, preferably ocrelizumab, and
   wherein the deviation between the NfL and GFAP levels of step a) with the scores or values of reference derived from the comparison step b) is used to predict a treatment outcome for said patient with Multiple Sclerosis when treated with the anti-CD20 antibody, preferably ocrelizumab.

In another preferred embodiment of the second aspect of the invention, the method is for predicting a treatment outcome with an anti-CD20 antibody, preferably ocrelizumab, in a patient who suffers from Multiple Sclerosis, comprising the steps of:
(a) quantifying NfL levels from an isolated blood or serum sample obtained from the patient after the anti-CD20 antibody, preferably ocrelizumab, treatment, preferably after 1, 2, 3, 6, 7, 8, 9, 10, 11, or 12 months after the first anti-CD20 antibody, preferably ocrelizumab, cycle, preferably about 3 months after the first anti-CD20 antibody, preferably ocrelizumab, cycle, and
(b) comparing said NfL levels of step a) with a reference value or reference score, wherein said reference value or reference score is calculated from a sample obtained from the patient before treatment (i.e., also referred herein, as baseline), and
   wherein if the value of step a) is lower, preferably statistically significantly lower, than the reference value or score, then the patient is identified as responsive to the treatment with the anti-CD20 antibody, preferably ocrelizumab. If the value of step a) is preferably not statistically significantly lower than the reference value or score, then the patient with multiple sclerosis is identified as not responsive if treated with the anti-CD20 antibody, preferably ocrelizumab.

In yet another preferred embodiment of the first aspect of the invention, the method is for predicting a treatment outcome with an anti-CD20 antibody, preferably ocrelizumab, in a patient who suffers from Multiple Sclerosis, comprising the steps of:
(a) quantifying NfL and GFAP levels from an isolated blood or serum sample obtained from the patient before treatment (i.e., also referred herein, as baseline), and
(b) comparing said NfL levels of step a) with a reference value or reference score, wherein said corresponding reference (value or score) is linked to a treatment outcome in patients having been treated with the anti-CD20 antibody, preferably ocrelizumab,
wherein if the sample of step a), obtained from the patient before treatment, comprises NfL and GFAP levels higher than the reference value/s or reference score/s, then the patient is identified as responsive to the treatment with the anti-CD20 antibody, preferably ocrelizumab.

It is noted that in the above embodiment the patient shall be preferably classified as responsive to the treatment with Ocrelizumab by using reference value/s or reference score/s of 10 pg/mL for sNfL and 140 pg/mL for sGFAP. That is, if the sample of step a), obtained from the patient before treatment, comprises sNfL levels greater than 10 pg/mL and sGFAP levels greater than 140 pg/mL, then the patient is classified as responsive to the treatment with ocrelizumab.

As used herein a patient responsive to the treatment with Ocrelizumab shall be understood as a patient classified herein as NEDA: patients who reached no evidence of disease activity (NEDA)-3 status at 12 months of ocrelizumab initiation or INFL: patients who had relapses and/or radiological activity but no disability progression at 12 months of ocrelizumab initiation. In both groups of patients, and as shown herein, Ocrelizumab treatment statistically significantly reduces the NfL levels after three months of treatment in comparison to a reference value or reference score calculated from a sample obtained from the patient before treatment (i.e., also referred herein, as baseline). That is, in these groups of patients the inflammatory activity caused by the disease is treated, reduced or ameliorated by the treatment with Ocrelizumab.

By "statistically significant" or "significant" is referred herein as the determination by an analyst that the results in the data are not explainable by chance alone. Statistical hypothesis testing is the method by which the skilled person makes this determination. This test provides a p-value, which is the probability of observing results as extreme as those in the data, assuming the results are truly due to chance alone. In an embodiment, a p-value is obtained from the comparison between the value of the amount of sNfL obtained and the reference value, and the difference is considered statistically significant if the p value is of 0 0.05, 0.01, 0.001 or lower. Different tests can be used to determine whether the differences between a value and a reference value are statistically different or not. Preferably, a Mann-Whitney U test or a Fisher's exact tests is used to obtain the p value according to the present invention.

In another preferred embodiment of the second aspect of the invention, the reference value or score is linked to a treatment outcome in patients having been treated Ocrelizumab and it is a cutoff as determined by a receiver operating characteristic (ROC) curve.

The present invention further provides, in a third aspect, a composition comprising Ocrelizumab for use in treating Multiple Sclerosis in groups (i) (patients at risk of having relapses and/or radiological activity but no disability progression at 12 months of ocrelizumab initiation) or (ii) (patients who reached no evidence of disease activity (NEDA) status at 12 months of ocrelizumab initiation) as identified by the first aspect of the invention. It is noted that, in the context of patients identified at risk of having relapses and/or radiological activity but no disability progression at 12 months of the ocrelizumab treatment initiation, if the value of step a) is not statistically significantly lower than the reference value or score as indicated in step c.i.2 of the first aspect of the invention, and the patient satisfies the criteria of step c.i.1 of the first aspect of the invention (both markers are increased), then the patient with multiple sclerosis is identified as responsive if treated with increased dosages and/or administration frequencies of Ocrelizumab. That is, for these types of patients' treatment with Ocrelizumab is deemed effective but should be fine-tune.

The present invention further provides in a fourth aspect a composition comprising Ocrelizumab for use in treating Multiple Sclerosis in a group of patients identified in accordance with the method of the first aspect of the invention as pertaining to groups (i) (patients at risk of having relapses and/or radiological activity but no disability progression at 12 months of ocrelizumab initiation) or (ii) (patients who reached no evidence of disease activity (NEDA) status at 12 months of ocrelizumab initiation). Again, it is noted that, in the context of patients identified at risk of having relapses and/or radiological activity but no disability progression at 12 months of the ocrelizumab treatment initiation, if the value of step a) is not statistically significantly lower than the reference value or score, and the patient satisfies the criteria of step c.i.1 of claim 1 (both markers are increased), then the patient with multiple sclerosis is identified as responsive if treated with increased dosages and/or administration frequency of Ocrelizumab. That is, for these types of patients' treatment with Ocrelizumab is deemed effective but should be fine-tune.

In a preferred embodiment of any of the previous aspects, MS is RRMS.

The present invention further provides, in a sixth aspect, a kit comprising means for quantifying NfL and/or GFAP levels in a blood or serum sample. It is noted that, optionally, the kit may further comprise any reagents (buffers, solutions, dyes...) needed to implement the method of any of aspects first or second of the invention.

Any one of the embodiments of the kit may further comprise Ocrelizumab. Preferably, a separate tube or recipient comprising Ocrelizumab.

The kits disclosed herein are based on the predictive power of the methods of the present invention. Reference values indicative of a responsive patient can be established using the method disclosed herein and the data disclosed in the Examples. In a preferred embodiment, a reference value which is indicative of a non-responsive patient and/or a reference value which is indicative for a responsive patient may be provided with the kit.

Further, it is to be interpreted that any embodiment of the kit of the sixth aspect can be used to implement any of the aspects third and fourth of the present invention. Thus, subject matter related to the kit is also related to the methods of the present invention. Thus, in a further aspect, the kit according to any one of the aforementioned kit embodiments may be used in any of the methods described herein.

The following examples merely illustrate the present invention.

### Examples

### METHODS

### Study Design

This multicenter prospective longitudinal study involved 100 patients diagnosed with MS according to the revised McDonald criteria, who consecutively began ocrelizumab treatment across 19 Spanish University Hospitals. Recruitment took place between February 2020 and March 2022 with patient follow-up until March 2023. Demographic, clinical, and radiological variables were collected at baseline. We prospectively followed the patients for 1 year, conducting clinical assessments every 6 months. We defined increases in the Expanded Disability Status Scale (EDSS) score as follows: at least 1.5 points for a baseline EDSS of 0, 1 point for a baseline EDSS of 1 to 5, and 0.5 points for a baseline EDSS of 5.5 or greater. In patients who increased EDSS at 1-year follow-up, maintenance of EDSS elevation was confirmed at 18 months of follow-up. Baseline MRI examinations were conducted 1 month before treatment initiation, following the clinical protocols established at each center. A second MRI study was conducted after 1 year of follow-up. We categorized the patients into three groups based on their response to ocrelizumab treatment. The NEDA group consisted of patients with no evidence of disease activity (NEDA-3), the INFL group comprised patients with relapses and/or radiological activity, and the PIRA group included patients with confirmed disability progression without relapses or new lesions in the MRI study. NEDA-3 was defined as the absence of relapses, MRI activity, and worsening disability.

### Sample Collection

Patient blood specimens were collected immediately prior to the initiation of ocrelizumab treatment, as well as at 3, 6, 9, and 12 months after that. The serum samples were sent to the Immunology Department of Hospital Ramón y Cajal (Madrid) and stored at -80°C until processing.

### Serum NfL and GFAP Quantification

Serum NfL and GFAP levels were quantified using the single molecule array (SIMOA) technique on an SR-X instrument (Quanterix, Lexington, MA). The NF-light Advantage Kit (Quanterix, Billerica, MA) and Serum GFAP Discovery Kit (Quanterix, Billerica, 100 MA) were used for the respective analyses, following the manufacturer's instructions.

### Statistical Analysis

Statistical analyses were conducted using GraphPad Prism 9.0 software (GraphPad Prism Inc., San Diego, CA). Categorical variables were analyzed using the χ2 104 test. The Wilcoxon matched-pairs test was employed to evaluate differences between samples collected at baseline and 3, 6, 9, and 12 months from the same patients. Inter-group comparisons were conducted using the Kruskal-Wallis and Mann-Whitney U tests. The Spearman test was used to examine correlations between sNfL and sGFAP values. Statistical significance was defined as p-values below 0.05.

### Standard Protocol Approvals, Registrations, and Patient Consents

The study was conducted at Ramón y Cajal University Hospital following the Strengthening the Reporting of Observational Studies in Epidemiology (STROBE) reporting guideline. This study received approval from the Ramón y Cajal University Hospital Clinical Research Ethics Committee. Prior to participation, written informed consent was obtained from each patient.

### RESULTS

This study included 100 MS patients (65 women, 65%), diagnosed at an average age of 41.2 ± 9.7 years (mean ± SD), who initiated ocrelizumab treatment in 19 Spanish hospitals. Of the participants, 24 patients (24%) were treatment-naive, while 76 (76%) switched from other disease-modifying treatments due to a lack of efficacy or safety concerns. After 1 year on ocrelizumab, 71 patients (71%) achieved NEDA-3 (NEDA), indicating no disease activity. Nineteen patients (19%) exhibited clinical and/or radiological activity (INFL), and ten patients (10%) experienced confirmed disability progression without disease activity (PIRA). Among the INFL patients, seven had relapses, 15 had at least one new T2 lesion on the 12-month MRI examination, and three of them displayed new gadolinium-enhancing lesions. Detailed baseline patient data can be found in Table 1. No significant differences were observed among the three groups regarding patient age or disease duration. However, the INFL group had a higher percentage of women (94.7%) compared to the NEDA group (56.3%) (p = 0.0022, Table 2).

Additionally, patients in the PIRA group exhibited a higher baseline EDSS score (p = 0.027, Table 2) than the INFL group. Furthermore, in patients with PIRA, the previous treatment was more frequently an oral drug than the INFL (p = 0.045) and NEDA (p = 0.0065) groups.

At baseline, INFL patients exhibited higher sNfL and sGFAP values than NEDA patients (p = 0.0003 and p = 0.027, respectively, Figure 1). There was a strong correlation between sNfL and sGFAP values in INFL patients (r = 0.75, p = 0.0003). In contrast, PIRA patients displayed low sNfL values, and although 50% of them had high sGFAP values, there was no correlation between sGFAP and sNfL levels. After 1 year of treatment, sNfL and sGFAP values decreased significantly in the NEDA group (p = 0.0005 and p = 0.000003, respectively) and the INFL group (p = 0.00004 and p = 0.005, respectively). However, no significant changes in either variable were observed in the PIRA patients (Figures 2A and 2B). We then examined the sNfL and sGFAP values at 3, 6, and 9 months of follow up to investigate the kinetics of changes (Figure 3).

After 3 months of ocrelizumab treatment, 88% of NEDA patients achieved sNfL values below 10 pg/mL, whereas only 36.8% of INFL patients did (p = 0.00001). The differences remained significant at six months (p = 0.005); by 9 months, no significant differences were observed between the two groups. These findings were consistent with the clinical data, as 71.4% of patients who experienced a relapse during treatment did so within the first 6 months following ocrelizumab administration. No changes in sNfL and sGFAP values were observed in the PIRA group. A reduction in sNfL levels was evident 3 months after initiating ocrelizumab in both the NEDA and INFL groups (Figure 3A). However, the decrease in sGFAP values occurred later. In NEDA patients with lower baseline GFAP values, a significant reduction was observed only 12 months after treatment initiation. In the inflammatory group, the reduction became significant at 9 months and remained consistent after 1 year (Figure 3B). During ocrelizumab treatment, no significant changes were observed in sNfL and sGFAP values in PIRA patients.

Using the sNfL values, we assessed the risk of developing inflammatory activity during the follow-up period (Figure 4). A 10 pg/mL cut-off value was applied, and we analyzed the results obtained from the baseline, 3-month, and 6-month samples (Figures 4A, 4B, and 4C, respectively). Among the values obtained, those obtained at 3 months after initiating ocrelizumab were determined as the most effective predictor of inflammatory status. Having sNfL values higher than 10 pg/mL at this time point increased the risk of experiencing inflammatory activity during the first year of treatment (p = 0.00001, Odds Ratio (OR) = 13.5; 95% confidence interval (CI): 4.1-47.4) (Figure 4B).

**Table 1. Baseline data and patient characteristics.**

| | |
|---|---|
| Variable | All patients (n=100) |
| Age (years) Median - [range] | 39.79 [22.06 - 65.59] |
| Sex (F/M) | 65/35 |
| Disease duration Median - [range] | 6.19 [0.10 - 30.84] |
| EDSS score Median - [range] | 2.0 [1.0 - 6.0] |
| sNfL Median - [range] | 7.33 [1.61 - 382.92] |
| sGFAP Median - [range] | 137.11 [46.51 - 1285.09] |
| Previous treatment - n (%) | |
| Naive | 24 (24%) |
| Platform | 11 (11%) |
| Orals | 48 (48%) |
| Monoclonal Ab | 17 (17%) |

| | |
|---|---|
| Abbreviations: F, Female; M, male; EDSS, Expanded Disability Status Scale; sNfL, serum neurofilament light chains; sGFAP, serum glial fibrillary acidic protein; n, number of patients; Ab, antibody. Naive: no previous treatment; Platform treatments included: interferon beta and glatiramer acetate; Oral drugs included: cladribine, dimethylfumarate, fingolimod and teriflunomide; Monoclonal Ab. included: alemtuzumab and natalizumab. | |

**Table 2. Differences among groups in baseline characteristics.**

| | NEDA (n=71) | INFL (n=19) | PIRA (n=10) | P value NEDA-INFL | P value NEDA-PIRA | P value INFL-PIRA |
|---|---|---|---|---|---|---|
| Age (years) | 40.92 | 37.27 | 45.93 | ns | ns | ns |
| Median - [range] | [22.06 - 65.59] | [25.24 - 61.36] | [28.86 - 62.70] | | | |
| Sex (F/M) | 40/31 | 18/1 | 7/3 | 0.0022 | ns | ns |
| Disease duration | 6.36 | 4.95 | 12.46 | ns | ns | ns |
| Median - [range] | [0.15 - 26.26] | [0.10 - 18.01] | [1.75 - 30.84] | | | |
| EDSS score | 2.5 | 2.0 | 3.5 | ns | ns | 0.027 |
| Median - [range] | [1.0 - 6.0] | [1.5 - 6.0] | [1.5 - 6.0] | | | |
| sNfL | 6.60 | 15.56 | 7.15 | 0.0003 | ns | ns |
| Median - [range] | [1.61 - 41.62] | [2.19 - 382.92] | [2.50 - 16.68] | | | |
| sGFAP | 122.41 | 196.55 | 154.50 | 0.027 | ns | ns |
| Median - [range] | [48.59 - 1285.09] | [85.29 - 1105.80] | [46.51-417.21] | | | |
| Previous treatment | | | | ns | 0.0065 | 0.045 |
| Naive | 17 (23.9%) | 7 (36.8%) | 0 (0%) | | | |
| Platform | 9 (12.7%) | 2 (10.5%) | 0 (0%) | | | |
| Orals | 29 (40.8%) | 9 (47.4%) | 10 (100%) | | | |
| Monoclonal Ab. | 16 (22.6%) | 1 (5.3%) | 0 (0%) | | | |

| | | | | | | |
|---|---|---|---|---|---|---|
| Abbreviations: F, Female; M, male; EDSS, Expanded Disability Status Scale; sNfL, serum neurofilament light chains; sGFAP, serum glial fibrillary acidic protein; n, number of patients; ns, non-significant; Ab, antibody. Naive: no previous treatment; Platform treatments included: interferon beta and glatiramer acetate; Oral drugs included: cladribine, dimethylfumarate, fingolimod and teriflunomide; Monoclonal Ab. included: alemtuzumab and natalizumab. | | | | | | |

## Claims

1. A method for monitoring or predicting the risk of disease activity derived from the treatment with Ocrelizumab in a patient who suffers from Multiple Sclerosis (MS), comprising the steps of:
a. quantifying NfL (neurofilament light chains) and GFAP (glial fibrillary acidic protein) levels from an isolated blood or serum sample taken from the patient,
b. comparing said NfL and GFAP levels of step a) with reference values or reference scores, wherein said corresponding reference (value or score) is linked to disease activity in patients having been treated with Ocrelizumab, and
c. identifying the patient as:
i. patients at risk of having relapses and/or radiological activity but no disability progression at 12 months of ocrelizumab initiation if
1. the sample of step a) is obtained from the patient before treatment, and the NfL and GFAP levels are higher than the reference value/s or reference score/s; and/or if
2. the isolated blood or serum sample is obtained from the patient after Ocrelizumab treatment, preferably after 1, 2, 3, 6, 7, 8, 9, 10, 11, or 12 months after the first Ocrelizumab cycle, preferably about 3 months after the first Ocrelizumab cycle, and the NfL levels of step a) when compared with a reference value or reference score, are lower, preferably statistically significantly lower, than the reference value or score, wherein said reference value or reference score is calculated from a sample obtained from the patient before treatment (i.e., also referred herein, as baseline);
ii. patients who reached no evidence of disease activity (NEDA) status at 12 months of ocrelizumab initiation if the criteria indicated at c.i.2 above are fulfilled and the levels of NfL in the isolated blood or serum sample obtained from the patient before treatment (i.e., also referred herein, as baseline) are below 10 pg/mL; and
iii. patients at risk of disability progression without inflammatory activity at 12 months of ocrelizumab initiation if the criteria indicated in c.i or c.ii are not fulfilled.

2. The method according to claim 1, wherein the reference value/s or reference score/s of c.i.1 are 10 pg/mL for NfL and 140 pg/mL for GFAP, so that if the sample of step a), obtained from the patient before treatment, comprises NfL levels greater than 10 pg/mL and GFAP levels greater than 140 pg/mL, the patient is classified as a patient at risk of having relapses and/or radiological activity but no disability progression at 12 months of ocrelizumab initiation.

3. A method for predicting a treatment outcome with Ocrelizumab in a patient who suffers from Multiple Sclerosis (MS), comprising the steps of:
a. quantifying NfL and/or GFAP levels from an isolated blood or serum sample taken from the patient, and
b. comparing said NfL and/or GFAP levels of step a) with a reference value or reference score,
wherein said corresponding reference (value or score) is linked to a treatment outcome in patients having been treated with Ocrelizumab, and
wherein the deviation between the NfL and/or GFAP levels of step a) with the score or value of reference derived from the comparison step b) is used to predict a treatment outcome for said patient with Multiple Sclerosis when treated with Ocrelizumab.

4. A method for predicting a treatment outcome with Ocrelizumab in a patient who suffers from Multiple Sclerosis (MS), comprising the steps of:
a. quantifying NfL and GFAP levels from an isolated blood or serum sample taken from the patient, and
b. comparing said NfL and GFAP levels of step a) with reference values or reference scores,
wherein said corresponding references (value or score) are linked to a treatment outcome in patients having been treated with Ocrelizumab, and
wherein the deviation between the NfL and GFAP levels of step a) with the scores or values of reference derived from the comparison step b) is used to predict a treatment outcome for said patient with Multiple Sclerosis when treated with Ocrelizumab.

5. A method for predicting a treatment outcome with Ocrelizumab in a patient who suffers from Multiple Sclerosis (MS), comprising the steps of:
a. quantifying NfL levels from an isolated blood or serum sample obtained from the patient after Ocrelizumab treatment, preferably after 1, 2, 3, 6, 7, 8, 9, 10, 11, or 12 months after the first Ocrelizumab cycle, preferably about 3 months after the first Ocrelizumab cycle, and
b. comparing said NfL levels of step a) with a reference value or reference score, wherein said reference value or reference score is calculated from a sample obtained from the patient before treatment (i.e., also referred herein, as baseline), and
wherein if the value of step a) is statistically significantly lower than the reference value or score, then the patient is identified as responsive to the treatment with Ocrelizumab, if the value of step a) is not statistically significantly lower than the reference value or score, and the patient satisfies the criteria of step c.i.1 of claim 1, then the patient with multiple sclerosis is identified as responsive if treated with increased dosages of Ocrelizumab.

6. A method for predicting a treatment outcome with Ocrelizumab in a patient who suffers from Multiple Sclerosis (MS), comprising the steps of:
a. quantifying NfL and GFAP levels from an isolated blood or serum sample obtained from the patient before treatment (i.e., also referred herein, as baseline), and
b. comparing said NfL levels of step a) with a reference value or reference score, wherein said corresponding reference (value or score) is linked to a treatment outcome in patients having been treated with Ocrelizumab,
wherein if the sample of step a), obtained from the patient before treatment, comprises NfL and GFAP levels higher than the reference value/s or reference score/s, then the patient is identified as responsive to the treatment with Ocrelizumab.

7. The method of claim 7, wherein the patient shall be preferably classified as responsive to the treatment with Ocrelizumab by using reference value/s or reference score/s of 10 pg/mL for NfL and 140 pg/mL for GFAP, so that if the sample of step a), obtained from the patient before treatment, comprises NfL levels greater than 10 pg/mL and GFAP levels greater than 140 pg/mL, then the patient is classified as responsive to the treatment.

8. The method of any one of claims 1 to 7, wherein MS is RRMS.

9. A composition comprising Ocrelizumab for use in treating Multiple Sclerosis, preferably RRMS, in groups c.i (patients at risk of having relapses and/or radiological activity but no disability progression at 12 months of ocrelizumab initiation) and/or c.ii (patients who reached no evidence of disease activity (NEDA) status at 12 months of ocrelizumab initiation) as identified by the method of claim 1 or 2.

10. A composition comprising Ocrelizumab for use in treating Multiple Sclerosis, preferably RRMS, in a group of patients identified in accordance with the method of claim 1 or 2 as pertaining to groups (i) (patients at risk of having relapses and/or radiological activity but no disability progression at 12 months of ocrelizumab initiation) and/or (ii) (patients who reached no evidence of disease activity (NEDA) status at 12 months of ocrelizumab initiation).

11. In vitro use of a kit comprising means for quantifying sNfL and/or sGFAP levels in a blood or serum sample to implement the method of any one of claims 1 to 7.
